# EUROPEAN PATENT SPECIFICATION

(11) **EP 3 253 866 B1**
(45) Date of publication and mention of the grant of the patent: **04.12.2024**
(21) Application number: 16703150.9
(22) Date of filing: 05.02.2016
(51) Int. Cl.: C12N 5/10, C12N 5/078, B82Y 5/00

(54) **PRIMARY HEMATOPOIETIC CELLS GENETICALLY ENGINEERED BY SLOW RELEASE OF NUCLEIC ACIDS USING NANOPARTICLES**
DURCH LANGSAME FREISETZUNG VON NUKLEINSÄUREN UNTER VERWENDUNG VON NANOPARTIKELN GENETISCH MANIPULIERTE PRIMÄRE HÄMATOPOETISCHE ZELLEN
CELLULES HÉMATOPOÏÉTIQUES PRIMAIRES MODIFIÉES GÉNÉTIQUEMENT PAR LIBÉRATION LENTE D'ACIDES NUCLÉIQUES À L'AIDE DE NANOPARTICULES

(30) Priority: 06.02.2015 DK 201570072
(43) Date of publication of application: 13.12.2017
(73) Proprietor: Cellectis, 75013 Paris (FR)
(72) Inventor: DELACOTE, Fabien, 75013 Paris (FR); DUCHATEAU, Philipe, 11500 Saint Louis et Parahou (FR)
(74) Representative: Zacco Denmark A/S
(86) International application number: PCT/EP2016/052552
(87) International publication number: WO 2016/124765

(56) References cited:
- WO-A1-2014/039523
- WO-A1-2014/184741
- WO-A1-2014/184741
- WO-A1-2014/191128
- US-A1- 2013 315 884
- US-A1- 2014 134 142
- NICOLE A MCNEER ET AL: "Nanoparticles Deliver Triplex-forming PNAs for Site-specific Genomic Recombination in CD34+ Human Hematopoietic Progenitors", MOLECULAR THERAPY, vol. 19, no. 1, 21 September 2010 (2010-09-21), pages 172 - 180, XP055177253, ISSN: 1525-0016, DOI: 10.1038/mt.2010.200
- PARK I Y ET AL: "Mannosylated polyethylenimine coupled mesoporous silica nanoparticles for receptor-mediated gene delivery", INTERNATIONAL JOURNAL OF PHARMACEUTICS, ELSEVIER BV, NL, vol. 359, no. 1-2, 9 July 2008 (2008-07-09), pages 280 - 287, XP022707727, ISSN: 0378-5173, [retrieved on 20080412], DOI: 10.1016/J.IJPHARM.2008.04.010
- KARISHMA T. MODY ET AL: "Mesoporous silica nanoparticles as antigen carriers and adjuvants for vaccine delivery", NANOSCALE, vol. 5, no. 12, 1 January 2013 (2013-01-01), United Kingdom, pages 5167, XP055273252, ISSN: 2040-3364, DOI: 10.1039/c3nr00357d
- DIWAN MANISH ET AL: "BIODEGRADABLE NANOPARTICLE MEDIATED ANTIGEN DELIVERY TO HUMAN CORD BLOOD DERIVED DENDRITIC CELLS FOR INDUCTION OF PRIMARY T CELL RESPONSES", JOURNAL OF DRUG TARGETING, HARWOOD ACADEMIC PUBLISHERS GMBH, DE, vol. 11, no. 8-10, 1 January 2004 (2004-01-01), pages 495 - 507, XP009081792, ISSN: 1061-186X, DOI: 10.1080/10611860410001670026
- YUFU WANG ET AL: "Modification of human BMSC with nanoparticles of polymeric biomaterials and plasmid DNA for BMP-2 secretion", JOURNAL OF SURGICAL RESEARCH, vol. 183, no. 1, 1 July 2013 (2013-07-01), pages 8 - 17, XP055177290, ISSN: 0022-4804, DOI: 10.1016/j.jss.2012.11.061

## Description

### Field of the invention

The present invention relates to a non-viral method for transfecting a hematopoietic cell which can be employed in immunotherapy. This method is based on the use of silica-based nanoparticle-biomolecule conjugates with high capacity for nucleic acid payload whereby their intracellular diffusion is slow and their toxicity much reduced. By using such a vectorization system, the targeted integration rate of exogenous nucleic acids into the cell is increased and their expression is extended. Nucleic acid to be transfected can encode, among others, chimeric antigen receptor and/or target-specific endonuclease. The expression of these exogenous nucleic acid sequences into the transformed hematopoietic cells improves their ability to fight infected or malignant cells. The present invention relates also to a method for transfecting/stimulating APCs. Furthermore, the present invention relates to transfected hematopoietic cell, which is obtainable according to the method of the invention and comprises a silica-based nanoparticle coated with a nucleic acid, which comprises a heterologous sequence encoding a rare-cutting endonuclease, as well as to pharmaceutical compositions comprising said cell. The transfected hematopoietic cell of the invention is particularly useful for the treatment of infected or malignant cells. The present invention is defined by the appended claims.

### Background of the invention

The feasibility of gene therapy is ultimately dependent on the availability of efficient gene vectors. Gene vectors are vehicles used to transport a desired genetic information encoded by a nucleic acid (DNA or RNA) into a target cell, and to have it expressed there. Viruses have evolved formidable solutions to this gene transfer problem. Consequently, genetically modified (recombinant) viruses rank among the most efficient vehicles known today for the transfer of foreign genetic information into cells. A multitude of viral species have been engineered as gene vectors, including retroviruses, adenoviruses, adeno-associated viruses, herpes simplex viruses, hepatitis viruses, vaccinia viruses and lentiviruses. In general, the genetic information required for the natural replicative cycle of the virus is removed from the viral genome and replaced by the gene(s) of interest which is/are supposed to exert some therapeutic effect in the case of gene therapy applications. Most recently, also replication-competent viruses have been used as gene transfer vehicles. Despite their efficacy, there are two main safety concerns for handling lentiviral plasmids: their potential to replicate and their potential oncogenesis.

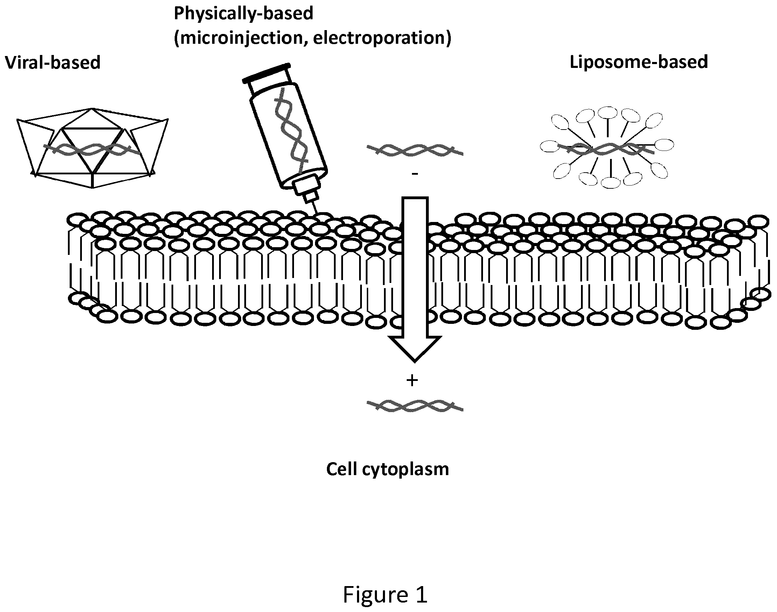

As an alternative to viral gene vectors, there are various methods of introducing foreign DNA into a eukaryotic cell: some rely on physical treatment (electroporation, cell squeezing, nanoparticles, magnetofection), other on chemical materials or biological particles (viruses) that are used as carriers (Figure 1). Non-viral, synthetic and half-synthetic vehicles for gene transfer have been developed over the last decade. Most of these non-viral vectors mimic important features of viral cell entry in order to overcome the cellular barriers to infiltration by foreign genetic material. Among these barriers are the plasma membrane, the membranes of internal vesicles such as endosomes and lysosomes and the nuclear membranes. Among the viral functions mimicked in non-viral vectors are the capability of receptor targeting, of DNA binding and compaction and of intracellular release from internal vesicles. These individual functions are represented in synthetic or half-synthetic modules which usually are assembled by electrostatic and/or hydrophobic interactions to form a vector particle. In order to systematically classify non-viral gene vectors according to their modular composition, the following nomenclature has been proposed (Feigner et al. 1997): Lipoplexes are assemblies of nucleic acids with a lipidic component, which is usually cationic. Gene transfer by lipoplexes is called lipofection. Polyplexes are assemblies of nucleic acids with an oligo- or polycationic entity. DNA complexes which comprise both classifications are called lipo-polyplexes or poly-lipoplexes. A huge variety of combinations of this general concept have been described. Examples include the classic cationic lipid-DNA complexes (Feigner and Ringold 1989), polycation-DNA complexes such as poly(lysine)-DNA (Wu and Wu 1987), poly(ethylene imine)-DNA (Boussif et al. 1995), poly(amido amine) dendrimer-DNA (Haensler and Szoka 1993), cationic peptide-DNA complexes (Plank et al. 1999), cationic protein-DNA complexes (histones, HMG proteins) (Zenke et al. 1990). Often such DNA complexes are further modified to contain a cell targeting or an intracellular targeting moiety and/or a membrane-destabilizing component such as an inactivated virus (Curiel et al. 1991), a viral capsid or a viral protein or peptide (Fender et al. 1997; Zhang et al. 1999) or a membrane-disruptive synthetic peptide (Wagner et al. 1992; Plank et al. 1994). Also, the nucleic acid to be transported has been enclosed in the aqueous lumen of liposomes (Nicolau and Cudd 1989), or polycation-condensed DNA is associated with a lipid membrane (Gao and Huang 1996; Li et al. 1998). The lipid membrane has also been composed to be a chimera of natural membranes derived from viruses or cells containing membrane proteins (HVJ liposomes for example (Kaneda 1998)]). Also bacteria (Grillot Courvalin et al. 1998) and phages (Poul and Marks 1999) have been described as shuttles for the transfer of nucleic acids into cells. Apart from these sophisticated vector compositions, also naked DNA is known to be a useful transfecting agent in certain applications (Wolff et al. 1990). The precipitation of DNA with divalent cations has been used successfully for the transfection of cultured cell lines for more than 10 years (calcium phosphate precipitation (Chen and Okayama 1988)]). Most recently, it has been found that calcium phosphate precipitation protocols are also useful in enhancing both viral and non-viral vector-mediated gene transfer (Fasbender et al. 1998). Vectors or naked DNA can also be formulated to achieve a sustained release or controlled release effect. For this purpose, DNA or vectors can be immobilized on/in or associated with carrier materials such as collagen (Bonadio et al. 1998), gelatin (Truong-Le et al. 1999) or fibrin glue. Also, DNA or vectors can be incorporated in micro- and nanoparticle formulations such as in copolymers like poly(lactic-co-glycolic acid) (PLGA) (Shea et al. 1999) and similar compositions or in nanoparticles prepared from chitosan (Roy et al. 1999).

Transfection techniques such as electroporation or liposomes (Figure 1) suffer from the limited amount of nucleic acid than can be introduced into the hematopoietic cells without being too toxic to them. Especially, primary hematopoietic cells show a marked resilience to transfection leading them to apoptosis, and thereby, a low yield of expressing cells is usually obtained (Emerson M et al, 2003, Molecular Therapy, 8, 646-653).

There is still a challenging need of a gene transfer method which can circumvent the drawbacks presented previously, in particular to allow the delivery of large amount of nucleic acid into primary hematopoietic cells with a reduced toxicity level, by allowing a slow diffusion profile. Furthermore, since this system is intended for *ex-vivo* and *in vivo* gene therapies, it is particularly recommended that it presents a good profile in terms of safety and an increased targeted integration rate.

### Summary of the invention

The inventors have sought and developed a non-viral transfection system based on silica-based nanoparticle-biomolecule conjugates with high capacity for nucleic acid payload whereby their intracellular diffusion is slow and their toxicity much reduced. Furthermore, the targeted integration rate is increased and the expression of the transfected nucleic acid extended.

According to the literature (for instance reviewed by Lian Jin, Xin Zeng, Ming Liu, Yan Deng and Nongyue He (2014) in Theranostics; 4(3):240-255) nanoparticles are believed to enter cells - for instance by endocytosis- as intact entities (Figure 1). Therefore, the nucleic acid which is transferred into cells has the particularity to not be under a naked form.

Here, the inventors found that silica-based nanoparticle-biomolecule conjugates were particularly suited to vectorize nucleic acids encoding rare cutting endonuclease to perform gene editing into hematopoietic cells, especially T-cells. It is particularly appropriate for two components rare-cutting endonucleases, where a non-specific nuclease such as Cas9 or Cpf1 has to be delivered along with a RNA or DNA guide that confers cleavage specificity. The nanoparticle allows the release of the two components simultaneously over an extended period of time.

Nanoparticles (NPs) used according to the present invention are silica-based NPs (SiNP), either under the mesoporous or the nanoporous form; the nucleic acid can be either encapsulated in the SiNPs or coated onto them. The nanoparticles (NPs) used according to the present invention are coated with cell penetrating peptides (CPPs) and/or ligand targeting molecule.

The engineered transfected immune cells as well as a pharmaceutical composition containing the same are particularly useful for therapeutic applications, such as for treating cancers, immune disorders or viral infections.

### Brief description of the figures

**Figure 1****:** Schematic representation of all the transfection systems known i.e. viral, physically-based and chemically-based whereby naked DNA is delivered into the cell.

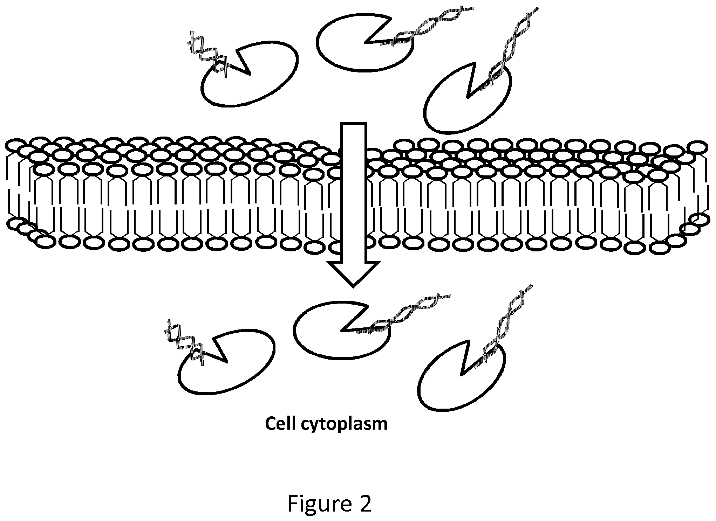

**Figure 2****:** Schematic representation of transfection by nanoparticles (NPs), whereby nucleic acid can enter into the cell under a conjugated form with those NPs i.e. not as a naked DNA.

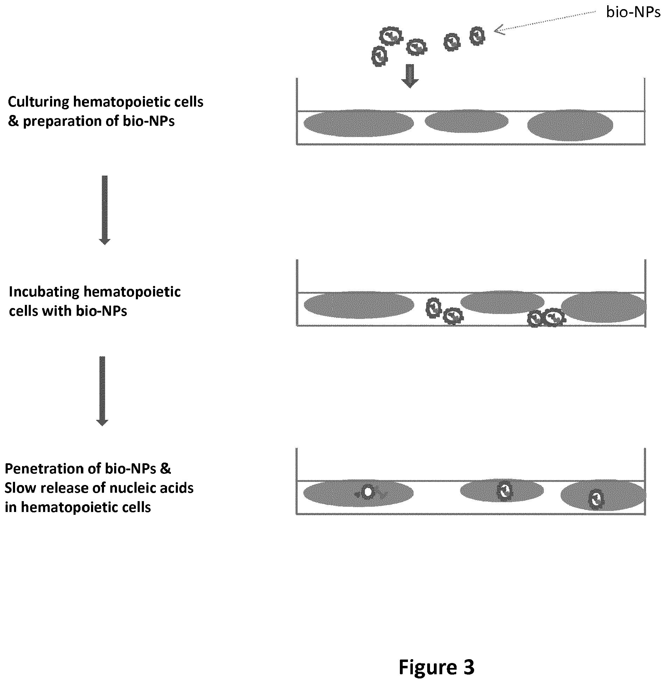

**Figure 3****:** Schematic representation of the transfection protocol. Here, are represented the step of culturing of primary hematopoietic cells and preparation of the nanoparticles-biomolecule conjugates (bio-NPs); the loading of the latter onto the said cells (for about 24 hours), and the incubation whereby the bio-NPs can be transfected into said cells.

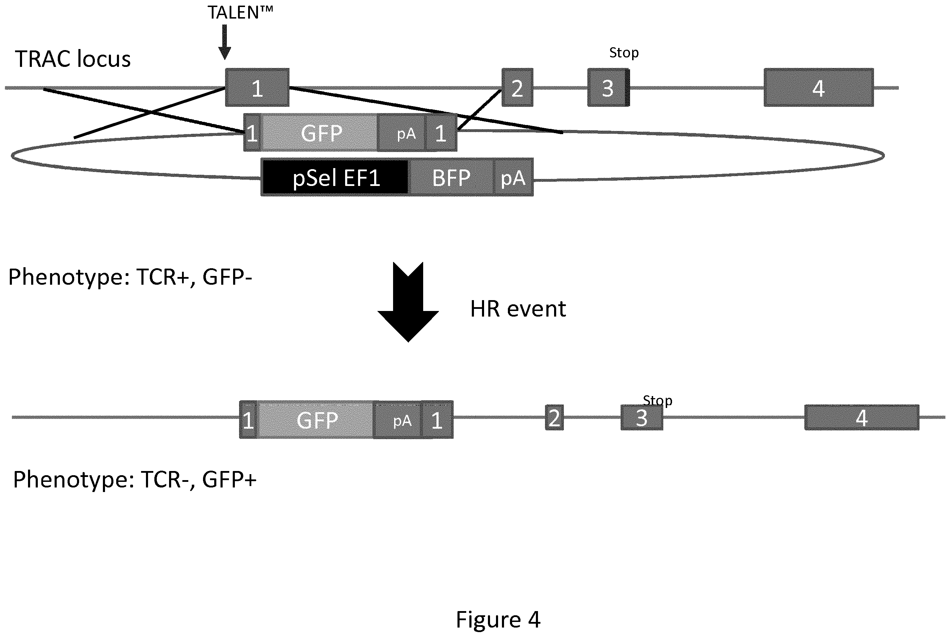

**Figure 4****:** Schematic representation of the homologous recombination (HR) of the GFP reporter gene within the TRAC locus of hematopoietic cell by using specific TALE-nuclease. The pSel EF1 plasmid with GFP& polyA tail (pA) shares 2 homologous sequences with the exon 1 of the TRAC locus from the TCR+ GFP- recipient cell. After a HR event, the GFP-pA integrates into the recipient cell, thus disrupting the TCR gene; the new phenotype obtained of this cell being then TCR- GFP+.

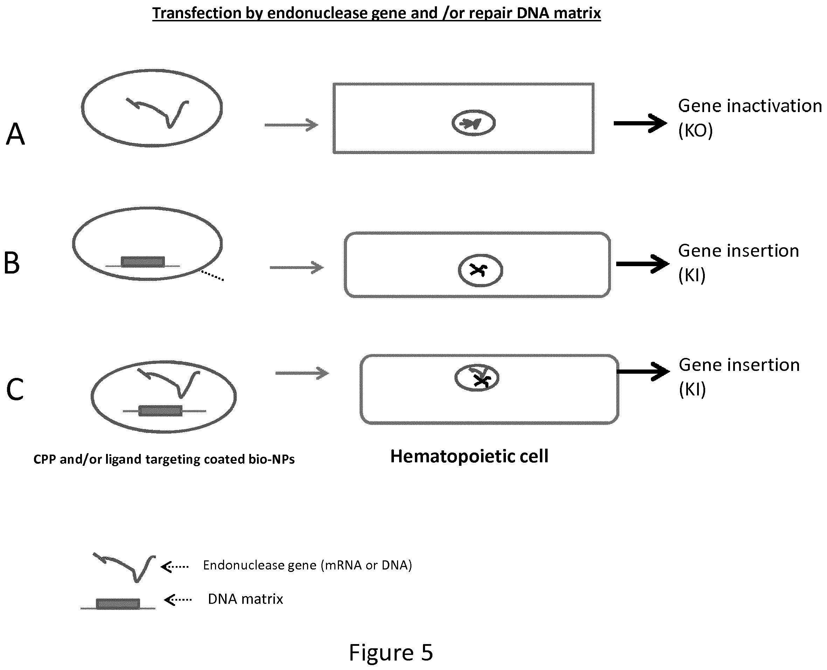

**Figure 5****:** Schematic representation of 3 possibilities of genome editing allowed by the NPs transfection method according to the invention. Fig 5A relates to the gene inactivation (KO) event when the hematopoietic cell is transfected by nanoparticle-biomolecule conjugates loaded with a target-specific endonuclease encoding mRNA or DNA -and coated with CPPs and/or ligand targeting molecules. Fig 5B and 5C relate both to the gene insertion (KI) event: Fig 5B corresponds to Example 2, wherein the matrix DNA is transfected by nanoparticle-biomolecule conjugates (bio-NPs) according to the invention, Fig 5C corresponds to Example 3, wherein both matrix DNA and target-specific endonuclease encoding mRNA or DNA are transfected by bio-NPs according to the invention.

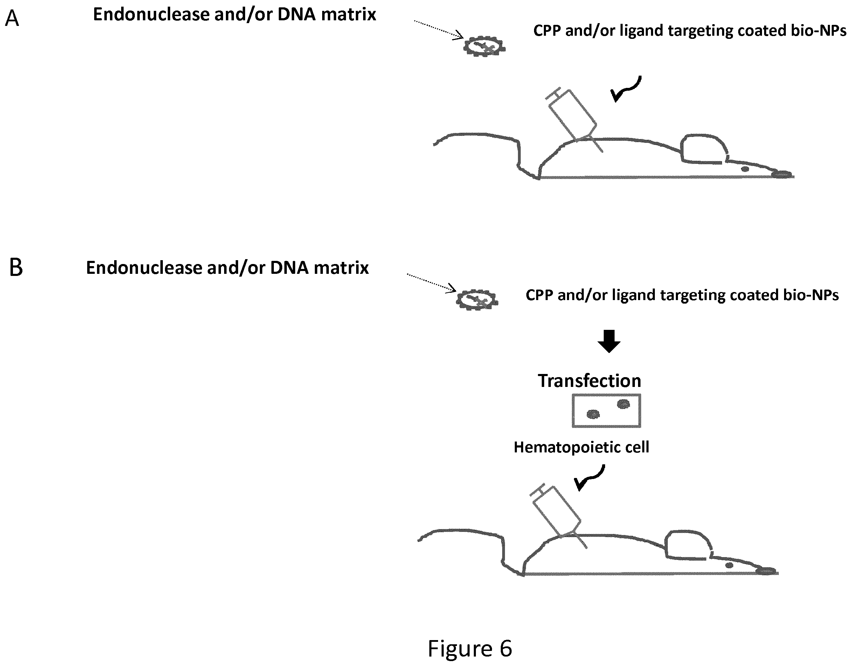

**Figure 6****:** Schematic representation of the in-vivo and ex-vivo treatments which are envisioned by the present invention for organ-specific gene editing in primary hematopoietic cells. In both cases, nanoparticle-biomolecule conjugates (bio-NPs) -coated with CPPs and/or ligand targeting molecules-can be loaded with target-specific endonuclease encoding mRNA or DNA and/or matrix DNA or chimeric antigen receptor (CAR). These bio-NPs are coated with targeting ligand/peptide to improve their targeting to the primary hematopoietic cells. Fig 6A corresponds to *in-vivo* treatment, wherein the transfection is performed directly by systemic injecting bio-NPs. Fig 6B corresponds to *ex-vivo* treatment, wherein the transfection is performed on beforehand isolated by bio-NPs.

### Detailed description of the invention

Unless specifically defined herein, all technical and scientific terms used have the same meaning as commonly understood by a skilled artisan in the fields of gene therapy, biochemistry, genetics, and molecular biology. All methods and materials similar or equivalent to those described herein can be used in the practice or testing of the present invention, with suitable methods and materials being described herein. In case of conflict, the present specification, including definitions, will prevail. Further, the materials, methods, and examples are illustrative only and are not intended to be limiting, unless otherwise specified.

The present inventors have sought and developed a method for a non-viral gene delivery whereby exogenous nucleic acid is slowly and gradually diffusing intracellularly and an increased targeted integration rate can be obtained. The main step of the process are schematized in the Figure 3.

The present invention relates a method of transfecting a primary hematopoietic cell with nucleic acids that comprise a heterologous sequence to be expressed into said cell or to be introduced into the genome, said method comprising more particularly several of the steps of:
a) Isolating;
b) culturing the primary hematopoietic cells in a condition where they can expand;
c) loading silica-based nanoparticle-biomolecule conjugates with said nucleic acids that comprise the heterologous sequence to be expressed into said cell or to be introduced into its genome; and
d) incubating said primary hematopoietic cells with said nanoparticle-biomolecule conjugates to have them penetrate the cells, and said nucleic acids are stabilized into primary hematopoietic cells over a period of time of more than two days;
wherein said nanoparticle-biomolecule conjugates are coated with cell penetrating peptides (CPPs) and/or ligand targeting molecule.

### Nanoparticles-biomolecule conjugate

According to the International Union of Pure and Applied Chemists (IUPAC) definition; the term "nanoparticle" corresponds to a sub-classification of ultrafine particle with lengths in two or three dimensions greater than 0.001 micrometer (1 nanometer) and smaller than about 0.1 micrometer (100 nanometers) and which may or may not exhibit a size-related intensive property. Because other phenomena (transparency or turbidity, ultrafiltration, stable dispersion, etc.) that extend the upper limit are occasionally considered, the use of the prefix nano is accepted for dimensions smaller than 500 nm.

As used in the present invention, the term *"biomolecule"* designs any type of nucleic acid: RNA, DNA or a mixture of both, modified or unmodified.

The term « *conjugate* » means in the scope of the present invention that nucleic acid is loaded onto the surface of the nanoparticle facilitating nanoparticle-molecule interaction and making them biocompatible. Conjugation can be achieved through intermolecular attractions between the nanoparticle and biomolecule (i.e. nucleic acid) such as covalent bonding, chemisorption or noncovalent interactions.

The present invention encompasses nanoparticle suitable to be loaded with heterologous nucleic acids in order to show an improved transfection by releasing gradually (and in particular of large amount of nucleic acids) said nucleic acids into hematopoietic cell.

According to the invention, the nanoparticles are silica-based nanoparticles coated with with cell penetrating peptides (CPPs) and/or ligand targeting molecule.

In a specific embodiment, said silica-based nanoparticles are mesoporous nanoparticles (MSNs). These spherical MSNs have the advantage to have tunable pore size and tunable outer particle diameter in the nanometer range. They can be prepared in a water/oil phase using organic templates method such as by simultaneous hydrolytic condensation of tetraorthosilicate to form silica and polymerization of styrene into polystyrene. An amino acid catalyst, octane hydrophobic-supporting reaction component, and cetyltrimethylammonium bromide surfactant can be used in the preparation process. The final step in the method may involve removal of the organic components by calcinations, yielding the mesoporous silica particles (Asep Bayu Dani Nandiyanto, Soon-Gil Kim, Ferry Iskandar, Kikuo Okuyama, (2009) "Synthesis of spherical mesoporous silica nanoparticles with nanometer-size controllable pores and outer diameters" Microporous and Mesoporous Materials, 120 (3), 447-453).

According to one embodiment, the nucleic acids are encapsulated in mesoporous nanoparticles (MSN). These nanoparticles of controlled diameter have very large and uniform regular pores are able to absorb a great amount of nucleic acids. They may be prepared by using a low temperature and a dual surfactant system such as Span 80 and Tween 80. To improve payloading, the external surface may be modified by addition of functionalized groups (i.e. aminopropyl groups) (Gao F, Botella P, Corma A, Blesa J, Dong L (2009) "Monodispersed mesoporous silica nanoparticles with very large pores for enhanced adsorption and release of DNA" J Phys Chem B; 113(6):1796-804).

According to another embodiment, the nucleic acids are coated onto mesoporous nanoparticles (MSN). To realize that by a common manner, cationic adjuncts may be applied to silica nanoparticles to electrostatically bind, protect from cleavage, and deliver DNA. As examples, silica particles (IPAST) or synthesized silica particles can be modified with either N-(2-aminoethyl)-3-aminopropyltrimethoxysilane or *N*-(6-aminohexyl)-3-aminopropyltrimethoxysilane. It is also possible to modify the external surface of these particles so that disulfide coupling chemistry can be used for immobilization of other molecules (i.e. oligonucleotides...) onto silica nanoparticles (Wagner E, Cotten M, Foisner R, Birnstiel ML. (1991) Proc Natl Acad Sci U S A.;88:4255-4259).

According to another embodiment, the silica-based nanoparticles are organic/inorganic silica hybrid nanoparticles which are coated with nucleic acids. This alternative does not require the use of solvent such as cyclohexane, their external organic groups prevent particle precipitation in aqueous systems, and their external surfaces can modified with targeting molecules. ORMOSIL (organically modified silane) such as *n*-octyl-triethoxysilane can aggregate in the form of normal micelles as well as reverse micelles in which the triethoxysilane moieties are hydrolyzed to form a hydrated silica network while the *n*-octyl groups are held together through hydrophobic interaction (Das S, Jain TK, Maitra A. J Colloid Interface Sci. 2002;252:82-88). Nanoparticles of various sizes (10-100 nm) can be produced according to Roy I, Ohulchanskyy TY, Bharali DJ, Pudavar HE, Mistretta RA, Kaur N, Prasad PN (2005). Proc Natl Acad Sci USA; 102:279-284. External surface amino functionalization allows these silica nanoparticles to electrostatically bind to negatively charged DNA and protect it from enzymatic degradation as shown by agarose gel electrophoresis. The same ORMOSIL nanoparticles may be functionalized with amino groups. MSNs may be coupled to mannosylated PEI (MPS) which are synthesized by a thiourea linkage reaction between the isothiocyanate group of α-D-mannopyranosylphenyl-isothiocyanate and the primary amine group of PEI. By MP functionalization render possible to target macrophage cells with mannose receptors and enhance transfection efficiency. These MPs are able to form complexes with DNA, protect against DNase I, and release DNA. Furthermore, they are acknowledged as having a low cytotoxicity suitable for gene delivery.

According to another embodiment, the nucleic acids are encapsulated into porous silica nanoparticle-supported lipid bilayers. These protocells (of about 100-150 nm in diameter), also called cell/silica composites (CSCs), can synergistically combine the features of mesoporous silica particles and liposomes to address targeted delivery. The high surface area and porosity of their nanoporous cores allow them to a much higher capacity when compared to the similarly sized liposomes. Furthermore, it is possible to graft on their surface number of targeting peptide for increasing their specificity to target cancerous cells. Such Si NPs are described in Ashley CE, Carnes EC, Phillips GK, Padilla D, Durfee PN, Brown PA. et al. (2011) "The targeted delivery of multicomponent cargos to cancer cells by nanoporous particle-supported lipid bilayers". Not Mater.;10:389-97).

The nanoporous silica particles that form the core of the protocell may be prepared, as previously from a homogenous mixture of water-soluble silica precursor(s) (i.e. TEOS) and amphipathic surfactant(s) (for instance CTAB, Abil EM 90), using either aerosol-assisted evaporation-induced self-assembly (EISA) or solvent extraction-driven self-assembly within water-in-oil emulsion droplets. Solvent evaporation or extraction concentrates the aerosol or emulsion droplets in surfactant(s), which directs the formation of periodic, ordered structures, around which silica assembles and condenses. Surfactants are removed via thermal calcination, which results in porous nanoparticles with well-defined, uniform pore sizes and topologies.

The lipid bilayer may comprise phosphatidylcholine molecules (such as 1,2-Dioleoyl-*sn*-glycero-3-phosphocholine ; 1,2-Dioleoyl-*sn*-glycero-3-phosphoethanolamine ; 1,2-Dipalmitoyl-*sn*-glycero-3-phosphocholine ; 1,2-Dipalmitoyl-*sn*-glycero-3-phosphoethanolamine), polyethyleneglycol derivative (i.e. 18.1 PEG -2000 PE), cholesterol molecules and crosslinker, for instance polyethylene glycol (PEG) or dithiobis (succinimidyl propionate).

As noted above, the nanoparticles (NPs) are coated by cell penetrating peptides (CPPs) and/or ligand targeting molecule.

According to the present invention, CPPs may be chosen amongst small molecules (such as folic acid, benzamides, Lex and Man LAM carbohydrates), homing peptides (for instance: EGF, CANF, angiopep-2), protein domains (such as from HER2, EGFR, PSA targets); antibodies (such as rituximab, trastuzumab, cetuximab), aptamers (short single-stranded nucleic acids RNA or DNA binding to targets such as PSMA, MUC1 or CD30 antigens), or multifunctional and chimeric approach (for instance, combination of aptamer with antibody). All these aspects are described in Adam D. Friedman, Sarah E. Claypool, and Rihe L (2013) "The Smart Targeting of Nanoparticles" Curr Pharm Des. 2013; 19(35): 6315-6329.

When utilizing nanoparticles for targeted delivery with any of the aforementioned ligands, it is often necessary to chemically modify the surface of the nanoparticles with an appropriate chemistry to introduce reactive moieties, thereby providing functional groups that can be conjugated to a targeting ligand of choice. It is important that the selective ligand has a functional group that can be used for conjugation as well. The conjugation of a targeting ligand to chemically modified nanoparticles will allow for selective delivery of the desired nanoparticle therapeutics. Most of the conjugation chemistries that are used to modify nanoparticles are covalent. Some of the most prevalent covalent reactions that are utilized in conjugating nanoparticles to targeting ligands include chemical reactions that use carbonyl reactive groups (i.e., carbonyl reacts with hydrazide or alkyoxyamine to form hydrazone or oxime bond), amine reactive groups (i.e., amine reacts with activated carboxylate or imidoester to form amide or amidine bond), sulfhydryl reactive groups (thiol reacts with maleimide, haloacetyl, pyridyl disulfide or gold surface, to form thioester, disulfide, or gold-thiol bond), and a type of orthogonal reaction known as Click Chemistry (i.e., azide reacts with phosphine or alkyne to form amide bond or triazole ring).

As a less preferred alternative, nanoparticles (NPs) may be multilayered and contain the nucleic acids in the core; NPs may contain one inner core-stabilizing interlayer comprises at least silica, chitosan, polyε-caprolactone, polyphosphoramidate (PPA), and one inner endosome-disrupting interlayer on the top of the core-stabilizing interlayer, said interlayer comprising at least a polycation.

This polycation can be chosen amongst polylysine, polyarginine, protamine, polyethylenimine, and histone. The outer layer of the NPs may comprise at least a biocompatible polymer (such as polyethylene glycol PEG). It may happen for the stability of the system that is required an additional layer comprising a polyanion, such as poly(D-glutamic acid).

The present also encompasses others compounds for their potential to improve gene delivery, such as histone H3 tail peptides or the papain-like cysteine protease Cathepsin B.

### Nucleic acid(s) to be transfected

Said nucleic acids comprise at least one heterologous sequence as non-naturally occurring in the recipient cell.

By *"nucleic acids",* it is meant at least one molecule of RNA or least one molecule of DNA or a mixture of both RNA and DNA. These nucleic acids can be modified or unmodified. For instance, a polyA tail can be added to mRNA to improve their nuclear export, translation and stability.

According to one embodiment, said nucleic acid(s) is at least one molecule of RNA

According to another embodiment, said nucleic acid(s) is at least one molecule of DNA.

According to another embodiment, said nucleic acids are a mixture of molecules of DNA and molecules of RNA.

Nucleic acid may encode, for example, a secreted hormone, enzyme, receptor, polypeptide, peptide or other protein of interest that is normally secreted. In one embodiment of the invention, the nucleic acids may optionally have chemical or biological modifications which, for example, improve the stability and/or half-life of such nucleic or which improve or otherwise facilitate protein production.

By the expression *"loading nanoparticle biomolecule conjugates with said nucleic acids",* it is meant that the nucleic acids are either encapsulated by the NPs or coats them.

According to one embodiment, said nucleic acids are tagged by a nuclear localization sequence (NLS). This NLS sequence tags a protein for import into the cell nucleus by nuclear transport. Typically, this signal consists of one or more short sequences of positively charged lysines or arginines exposed on the protein surface. As examples, NLSs may be the sequence PKKKRKV in the SV40 Large T-antigen, KR[PAATKKAGQA]KKKK from nucleoplasmin, K-K/R-X-K/R sequence, acidic M9 domain of hnRNP A1, the sequence KIPIK in yeast transcription repressor Matα2, and the complex signals of U snRNPs (Görlich D et al, 1997, "Nuclear protein import". Current Opinion in Cell Biology 9 (3): 412-9); Lusk CP, Blobel G, King MC (May 2007). "Highway to the inner nuclear membrane: rules for the road". Nature Reviews Molecular Cell Biology 8 (5): 414-20).

According to one embodiment, the nucleic acids are encoding a chimeric antigen receptor (CAR).

These artificial (engineered) T cell receptors are under investigation as a therapy for cancer, using a technique called adoptive cell transfer. T cells are removed from a patient and modified by grafting the specificity of a monoclonal antibody, so that they express receptors specific to the particular form of cancer. The immune cell (i.e. T cells), which can then recognize and kill the cancer cells, are reintroduced into the patient.

CARs are synthetic receptors consisting of a targeting moiety that is associated with one or more signaling domains in a single fusion molecule. In general, the binding moiety of a CAR consists of an antigen-binding domain of a single-chain antibody (scFv), comprising the light and variable fragments of a monoclonal antibody joined by a flexible linker. Binding moieties based on receptor or ligand domains have also been used successfully. The invention encompasses first generation CARs wherein signaling domains for are derived from the cytoplasmic region of the CD3zeta or the Fc receptor gamma chains. The invention covers also second and third generations, which allow prolonged expansion and anti-tumor activity *in vivo.* For these CARs, signaling domains from co-stimulatory molecules, as well as transmembrane and hinge domains have been added to form CARs.

According to one embodiment, said CAR is a single-chain CAR.

They may be designed according to single-chain as well defined in the prior art, such as in US7446190, WO2008/121420, US8252592, US20140024809, WO2012/079000, WO2014153270, WO2012/099973, WO2014/011988, WO2014/011987, WO2013/067492, WO2013/070468, WO2013/040557, WO2013/126712, WO2013/126729, WO 2013/126726, WO2013/126733, US8399645, US20130266551, US20140023674, WO2014039523, US7514537, US8324353, WO2010/025177, US7446179, WO2010/025177, WO2012/031744, WO2012/136231A1, WO2012/050374A2, WO2013074916, WO/2009/091826A3, WO2013/176915 or WO/2013/059593.

According to another embodiment, said CAR is a multichain CAR. Examples of multi-chain architectures of CAR are more particularly disclosed in WO2014039523.

According to another embodiment, the CAR comprises at least a CD3 zeta signaling domain and a 4-1BB co-stimulatory domain.

According to another embodiment, the CAR is specific to a cell surface antigen chosen amongst C38, CD123 or CS1.

According to another embodiment, the nucleic acids are encoding a target-specific endonuclease.

In case of genome editing, DNA is inserted, replaced, or removed from a genome using artificially engineered nucleases, or "molecular scissors." The nucleases create specific double-stranded break (DSBs) at desired locations in the genome, and harness the cell's endogenous mechanisms to repair the induced break by natural processes of homologous recombination (HR) and non-homologous end-joining (NHEJ). For doing so, engineered nucleases such as zinc finger nucleases (ZFNs), Transcription Activator-Like Effector Nucleases (TALENs), the CRISPR/Cas system, and engineered meganuclease re-engineered homing endonucleases are routinely used for genome editing.

According to another preferred embodiment, the rare-cutting endonuclease is Cas9, Cpf1, , TALEN, ZFN, or a homing endonuclease. Also, it may be convenient to engineer using DNA-guided Argonaute interference systems (DAIS). Basically, said Argonaute (Ago) protein is heterologously expressed from a polynucleotide introduced into said cell in the presence of at least one exogenous oligonucleotide (DNA guide) providing specificity of cleavage to said Ago protein to a preselected locus.

The TALEN and Cas9 systems are respectively described in WO 2013/176915 and WO 2014/191128.

The Zinc-finger nucleases (ZFNs) are initially described in Kim, YG; Cha, J.; Chandrasegaran, S. ("Hybrid restriction enzymes: zinc finger fusions to Fok I cleavage domain" (1996). Proc Natl Acad Sci USA 93 (3): 1156-60).

Cpf1 is class 2 CRISPR Cas System described by Zhang et al. (Cpf1 is a single RNA-guided Endonuclease of a Class 2 CRIPR-Cas System (2015) Cell;163:759-771).

The argonaute (AGO) gene family was initially described in Guo S, Kemphues KJ. ("par-1, a gene required for establishing polarity in C. elegans embryos, encodes a putative Ser/Thr kinase that is asymmetrically distributed" (1995) Cell;81(4):611-20).

The use of the CRISPR/Cas9, CRISPR/Cpf1 or the Argonaute genome-editing systems is particularly adapted to the transfection method of the invention by using bio-NPs. This can be performed by introducing into the cell guide-RNAs and a nucleic acid sequence coding for Cas9 nickase, so that they form a complex able to induce a nick event in double-stranded nucleic acid targets in order to cleave the genetic sequence between said nucleic acid targets.

In certain embodiments, the invention provides nanoparticle formulation comprising one or more guide RNAs that are delivered in vitro and/or ex vivo in the context of the CRISPR- Cas system.

In certain embodiments, it may be useful to deliver the guide RNA-nanoparticle formulations separately from the Cas9. In such an instance a dual-delivery system is provided such that the Cas 9 may be delivered via a vector and the guide RNA is provided in a nanoparticle formulation, where vectors are considered in the broadest sense simply as any means of delivery, rather than specifically viral vectors. Separate delivery of the guide RNA-nanoparticle formulation and the Cas 9 may be sequential, for example, first Cas9 vector is delivered via a vector system followed by delivery of sgRNA-nanoparticle formulation) or the sgRNA-nanoparticle formulation and Cas9 may be delivered substantially contemporaneously (i.e., co-delivery). Sequential delivery may be done at separate points in time, separated by days, weeks or even months.

In certain embodiments, multiple guide RNAs formulated in one or more delivery vehicles (e.g., where some guide RNAs are provided in a vector and others are formulated in nanoparticles) may be provided with a Cas9 delivery system.

In certain embodiments, the Cas9 is also delivered in a nanoparticle formulation. In such an instance the guide RNA-nanoparticle formulation and the Cas9 nanoparticle formulation may be delivered separately or may be delivered substantially contemporaneously (i.e., co- delivery). Sequential delivery could be done at separate points in time, separated by days, weeks or even months.

In certain embodiments, nanoparticle formulations comprising one or more guide RNAs are adapted for delivery in vitro, ex vivo or in vivo in the context of the CRISPR-Cas system to different target genes, different target hematopoietic cells. Multiplexed gene targeting using nanoparticle formulations comprising one or more guide RNAs are also contemplated.

In an embodiment, a nanoparticle formulation comprising one or more components of the CRISPR-Cas system is provided.

In another embodiment, a gRNA-nanoparticle formulation comprising one or more guide RNAs is provided.

The invention accordingly comprehends admixing sgRNA, Cas9 or Cpf1 protein and components that form a particle; as well as particles from such admixing. Typically, Cas9 protein and sgRNA targeting the gene EMX1 or the control gene LacZ were mixed together at a suitable, e.g.,3:1 to 1:3 or 2:1 to 1:2 or 1:1 molar ratio, at a suitable temperature, e.g., 15-30C, e.g., 20-25C, e.g., room temperature, for a suitable time, e.g., 15-45, such as 30 minutes, advantageously in sterile, nuclease free buffer, e.g., 1X PBS. Separately, particle components such as or comprising: a surfactant, e.g., cationic lipid, e.g., 1,2-dioleoyl-3-trimethylammonium-propane (DOTAP); phospholipid, e.g., dimyristoylphosphatidylcholine (DMPC); biodegradable polymer, such as an ethylene-glycol polymer or PEG, and a lipoprotein, such as a low-density lipoprotein, e.g., cholesterol were dissolved in an alcohol, advantageously a C₁₋₆ alkyl alcohol, such as methanol, ethanol, isopropanol, e.g., 100% ethanol. The two solutions were mixed together to form particles containing the Cas9-sgRNA complexes.

Accordingly, the invention comprehends admixing sgRNA, Cas9 protein and components that form a particle, e.g., comprising admixing an sgRNA and Cas9 protein mixture with a mixture comprising or consisting essentially of or consisting of surfactant, phospholipid, biodegradable polymer, lipoprotein and alcohol, and such a method to form particles containing the sgRNA and Cas9 protein, and particles therefrom.

In a preferred embodiment, particles containing the Cas9-sgRNA complexes may be formed by mixing Cas9 protein and one or more sgRNAs together, preferably at a 1:1 molar ratio, enzyme: guide RNA. Separately, the different components known to promote delivery of nucleic acids (e.g. DOTAP, DMPC, PEG, and cholesterol) are dissolved, preferably in ethanol. Typically, the two solutions are mixed together to form particles containing the Cas9-sgRNA complexes. After the particles are formed, Cas9-sgRNA complexes may be transfected into cells (e.g. HSCs). Bar coding may be applied.

According to one particular embodiment, CRISPR-Cas9 or CRISPR-Cpf1 based nanoparticle-biomolecule conjugate (NPBC) comprises at least a single stranded DNA partially complemented to sgRNA, a Cas9or Cpf1 protein, and a cationic polymer. The single stranded DNA (ssDNA) is preferably a long DNA molecule that can hybridize many copies of one or different sgRNAs. This DNA molecule is like "loaded" with sgRNA, which are thereby stabilized and less prompt to degradation. The loaded DNA molecule forms like a bundle, which is included in the nanoparticle. It was earlier reported that such DNA molecules could encapsulate the chemotherapeutic agent doxorubicin and control its release based on the environmental conditions.

Said ssDNA is designed to comprise many sequences that are partially complementary to 5' end of the sgRNAs, the rationale being that the complementary sequence would promote base pairing leading to a strong but reversible interaction. Preferably, ssDNA is designed to have sequences that can hybridize sgRNA between 1 to 50 nucleotides long, more preferably between 5 to 25 nucleotide longs, and even more preferably between 10 to 17 nucleotides long. Such ssDNA can be synthesized, for instance, by rolling circle amplification (RCA). This method aims the palindromic sequences encoded to drive the self-assembly of nanoparticles. Such technique is described, for instance, in Ali MM, Li F, Zhang Z, Zhang K, Kang DK, Ankrum JA, Le XC, Zhao W (2014) "Rolling circle amplification: a versatile tool for chemical biology, materials science and medicine". Chem Soc Rev. 43(10):3324-41.

Preferably, said cationic polymer, which induces endosomal escape, is polyethylenimine (PEI).

Preferably, a nuclear-localization-signal peptide is fused to Cas9 in order to allow Cas9/sgRNA complex to be transported into the nucleus.

Preferably, the stoichiometry of the Cas9/sgRNA complex is comprised between 5:1 and 0.5: 1 and more preferably is 1:1.

According to a preferred embodiment, CRISPR-Cas9 based nanoparticle-biomolecule conjugate (NPBC) comprises at least a ssDNA loaded with a single guide RNA (sgRNA), a Cas9 protein and polyethylenimine (PEI); wherein the stoichiometry of the Cas9/sgRNA complex is 1:1, and ssDNA is designed to have between 10 to 17 nucleotides complementary to the sgRNA.

In a specific embodiment of the invention, the CRISPR-Cas9 based nanoparticle-biomolecule conjugate (NPBC) is aimed to genetically inactivate at least one gene selected from the group consisting of CD52, GR, TCR alpha and TCR beta, or drug resistance gene such as dCK gene or phosphoribosyl transferase (HPRT) gene.

In another specific embodiment of the invention, the CRISPR-Cas9 based nanoparticle-biomolecule conjugate (NPBC) is aimed to genetically inactivate at least one gene acting as immune checkpoint, listed in this table 1 of the application WO2013176915, involved into co-inhibitory receptor function, cell death, cytokine signaling, arginine tryptophan starvation, TCR signaling, Induced T-reg repression, transcription factors controlling exhaustion or anergy, and hypoxia mediated tolerance.

In another embodiment, the genetic modification step of the method relies on the inactivation of more than two genes. The genetic modification is preferably operated ex-vivo using at least two RNA guides targeting the different genes.

According to a one embodiment, the CRISPR-Cas9 based nanoparticle-biomolecule conjugate (NPBC) comprises at least one sgRNA targeting the respective 20 bp sequences (5' to 3') in the CD52 gene (SEQ ID NO. 6)

According to a preferred embodiment, the CRISPR-Cas9 based nanoparticle-biomolecule conjugate (NPBC) comprises at least one sgRNA targeting the respective 20 bp sequences (5' to 3') in the TCRalpha gene (SEQ ID NO.7).

According to a specific embodiment, the CRISPR-Cas9 based nanoparticle-biomolecule conjugate (NPBC) comprises at least one sequence encoding the Cas9 from S. pyogenes. Such sequence encoding the Cas9 may be found by instance in the application WO2014191128; as synthesized de novo (GeneCust) and flanked by 3xNLS and a HA tag at the C-terminus (pCLS22972 of SEQ ID NO.53 in the above PCT application).

According to another embodiment, nucleic acids from both CAR and target-specific endonuclease are used to transfect/express primary hematopoietic cells.

### Primary hematopoietic cell

Primary cells are cultured directly from a subject; they are to be differentiated from established cell lines which are not contemplated in the scope of the present invention. Hematopoietic cells correspond to lymphoid cells such as T-cells, B-cells, NK-cells and to myeloid cells such as monocytes, macrophages, neutrophils, basophils, eosinophils, erythrocytes, megakaryocytes/platelets and dendritic cells.

According to one embodiment, said hematopoetic cells are T-cells.

According to one embodiment, said hematopoetic cells are previously isolated from donors.

According to one embodiment, said hematopoetic cells are previously isolated from a patient.

### Method of transfection

The present invention relates more particularly to a method of transfecting a primary hematopoietic cell with nucleic acids, at least one which encodes for a rare-cutting endonuclease, to be expressed into said cell or to be introduced into its genome, said method comprising the steps of:
a) isolating hematopoietic cells;
b) culturing the hematopoietic cells in a condition where they can expand;
c) loading nanoparticle-biomolecule conjugates with nucleic acids, at least one of which encodes a rare-cutting endonuclease to be expressed into said cell or to be introduced into its genome;
d) incubating said hematopoietic cells with said nanoparticle-biomolecule conjugates to have them penetrate the cells;
wherein the nanoparticle-biomolecule conjugates are silica-based nanoparticles which are coated with cell penetrating peptides (CPPs) and/or ligand targeting molecule.

By *"transfection"* means that the nucleic acids (negatively-charged substance) are transferred into the cell and is located, at the end of the process, inside said cell. The term is used here for non-viral methods of introducing nucleic acids in primary hematopoietic cells. All kind of genetic material (such as supercoiled plasmid DNA or siRNA constructs) may be transfected. Such depicted in Figure 4, several mechanisms of transfection are encompassed within the present invention, depending of the objective to achieved (gene inactivation or gene insertion). In these methods of transfection, nanoparticles-biomolecule conjugates (bio-NPS) can contain endonuclease gene and/or DNA matrix.

This time of incubation needs to be adapted to sufficiently depending of the nucleic acid to be transfected, it should be long enough to allow a slow release of the nanoparticle-biomolecule conjugates into the hematopoietic cells but not in excess to prevent toxicity of the nucleic acid and/or off-site cleavages by endonuclease encoded by the transfected nucleic acid.

According to a preferred embodiment, said step d) of incubation said hematopoietic cells with said nanoparticle-biomolecule conjugates is performed between 1 hour and 2 days.

According to a preferred embodiment, said step d) of incubation said hematopoietic cells with said nanoparticle-biomolecule conjugates is performed for at least 24 hours.

The present invention relates more particularly to a method of transfecting a hematopoietic cell to by using nanoparticle-biomolecule, wherein the period of time of release of nucleic acids loaded is comprised between 2 and 14 days, preferably between 4 and 10 days, more preferably between 4 and 7 days. As nucleic acid is generally toxic to most cells, its slow release or diffusion into these cells allows a decrease of its toxicity. Furthermore, the presence of targeting ligand on the surface of the NPs allows them to target in a more efficient manner the cells, whereas the presence of NLS sequence in the nucleic acids allow them to enter into the nucleus.

According to one embodiment, the present invention relates to a method of transfecting a hematopoietic cell to by using nanoparticle-biomolecule, wherein said nucleic acids persist into said hematopoietic cells over a period of time of more than two days.

According to a preferred embodiment, the present invention relates to a method of transfecting a hematopoietic cell to by using nanoparticle-biomolecule, wherein said nucleic acids persist into said hematopoietic cells between 2 and 14 days, preferably between 4 and 10 days, more preferably between 4 and 7 days.

By the term "persist", it is meant that the nucleic acids that are released from the nanoparticle conjugate are found at detectable levels in the cell's cytoplasm, preferably at least 5%, more preferably 10 % of the amount of nucleic acid borne by the nanoparticle is detectable.

The stabilization of nucleic acids is monitored by classical gene expression techniques to examine mRNA expression levels or differential mRNA expression. Transfection can be examined by any appropriate method, for example by measuring the expression of said gene or by measuring the concentration of the expressed protein. Suitable methods of transfection, measuring the expression of said gene or measuring the concentration of the expressed protein, or methods for detecting the viability of the cell (such as MTT assays) are well known to the person skilled in the art (see, e.g., Cell Biology. A Laboratory Handbook: vol. 4, Kai Simons, J. Victor Small, Tony Hunter, Julio E. Celis, Nigel Carter, (2005) Elsevier Ltd, Oxford; Auflage: 3rd ed. Literature). Some examples of these techniques are reporter gene, northern blotting, western blotting, fluorescent in situ hybridization (FISH), reverse transcription polymerase chain reaction (RT-PCR), serial Analysis of Gene Expression (SAGE), DNA microarray, RNA sequencing, tiling arrays. This monitoring of nucleic acid to be transfected by the bio-NPs of the present invention can be performed in parallel with that done using other transfection techniques. The comparison of the levels of transfection/expression can be therefore be assessed from those different techniques.

The transfection by nanoparticles may be performed by the addition of transfection agents such as lipid based or N-TER peptide (Sigma) reagents to boost the ability to transfect much recalcitrant eukaryotic cell types such as immune cells. Serum-free medium is preferred.

The protocol used for the transfection step is followed according to the manufacturer's recommendations.

According to one embodiment, an additional incubation is performed with hyaluronidase and/or collagenase. The use of these enzymes was shown to improve transfection efficacy in some cells such as chondrocyte (J. Haag, R. Voigt, S. Soeder, , (2009), "Efficient non-viral transfection of primary human adult chondrocytes in a high-throughput format" Osteoarthritis and Cartilage ; 17(6) : 813-817).

According to one embodiment, the transfection method comprises, after the incubation of primary hematopoietic cells with nanoparticles-biomolecule conjugates (bio-NPS), a step of purification of primary hematopoietic cells which have expressed or integrated into their genome said heterologous nucleic sequence.

### Targeted recombination

DNA integration into the genome occurs through the distinct mechanisms of homologous recombination or non-homologous end joining (NHEJ). However, NHEJ is an imperfect repair process that often results in changes to the DNA sequence at the site of the cleavage.

The present disclosure relates to a method for enhancing targeted integration in hematopoietic cell comprising the step of:
a) transfecting a hematopoietic cell according to the transfection such as described previously with nanoparticles coated with a matrix comprising an exogenous nucleic acid and a nucleic acid sequence homologous to a genomic sequence;
b) after incubation, selecting the primary hematopoietic cells where said exogenous nucleic acid has been integrated into the genome.

The targeted integration events by HR or NHEJ with or without repair DNA matrix are shown in Figure 5.

The aim is to develop a transfection method which gives an improved targeted integration rate.

According to one example, said nucleic acids are at least one repair DNA matrix that can be integrated through homologous recombination (HR) at a genome site.

According to another example, said nucleic acids are at least one repair DNA matrix that can be integrated through non-homologous end-joining (NHEJ) at a genome site.

According to another example, at least one said nanoparticles are coated with nucleic acids encoding a rare-cutting endonuclease.

According to another example, the nanoparticles are loaded with both or either nucleic acids a least one matrix inducing targeted integration at a genome site and a nucleic acid expressing a rare-cutting endonuclease targeting said genome site.

The introduction into cells at least one exogenous nucleic acid comprising at least a sequence homologous to a portion of the target nucleic acid sequence(s), such that targeted integration occurs between the target nucleic acid sequence(s) and the exogenous nucleic acid(s).

Said exogenous nucleic acid(s) usually comprises first and second portions which are homologous to region 5' and 3' of the target nucleic acid sequence, respectively. Said exogenous nucleic acid may also comprise a third portion positioned between the first and the second portion which comprises no homology with the regions 5' and 3' of the target nucleic acid sequence. Following cleavage of the target nucleic acid sequence, a targeted integration event is stimulated between the target nucleic acid sequence and the exogenous nucleic acid. Preferably, homologous sequences of at least 50 bp, preferably more than 100 bp and more preferably more than 200 bp are used within said donor matrix. Therefore, the exogenous nucleic acid(s) is preferably from 200 bp to 6000 bp, more preferably from 1000 bp to 2000 bp. Indeed, shared nucleic acid homologies are located in regions flanking upstream and downstream the site of the break and the nucleic acid sequence to be introduced should be located between the two arms.

Within the scope of the present disclosure, is encompassed a method for inactivating a gene into a hematopoietic cell, comprising the steps of:
a) transfecting a hematopoietic cell according to the transfection method such as described previously, with nanoparticles coated with nucleic acids encoding a rare-cutting endonuclease targeting a genomic locus;
b) after incubation, selecting the primary hematopoietic cells where said genomic locus has been interrupted. By inactivating a gene, it is intended that the gene of interest is not expressed in a functional protein form. In particular embodiment, the genetic modification of the method relies on the expression, in provided cells to engineer, of one rare-cutting endonuclease such that said rare-cutting endonuclease specifically catalyzes cleavage in one targeted gene thereby inactivating said targeted gene. The nucleic acid strand breaks caused by the rare-cutting endonuclease are commonly repaired Mechanisms involve rejoining of what remains of the two DNA ends through direct re-ligation (Critchlow and Jackson 1998) or via the so-called microhomology-mediated end joining (Ma, Kim et al. 2003).

According to one specific example, the method allows the inactivation of gene chosen amongst CD52, GR, TCR alpha and TCR beta, or drug resistance gene such as dCK gene or phosphoribosyl transferase (HPRT) gene.

By transfection, the exogenous nucleic acid(s) successively comprises a first region of homology to sequences upstream of said cleavage, a sequence to inactivate one selected targeted and a second region of homology to sequences downstream of the cleavage.

Gene inactivation can be done at a precise genomic location targeted by a specific endonuclease such a TALE-nuclease, wherein said specific endonuclease catalyzes a cleavage and wherein said exogenous nucleic acid(s) successively comprising at least a region of homology and a sequence to inactivate one selected targeted gene which is integrated by targeted integration

According to another example, there is a method for inserting a protein coding sequence at a particular locus by using the transfection method described above. As examples, are the drug-resistance genes such as Dihydrofolate reductase (DHFR), ionisine-5'- monophosphate dehydrogenase II (IMPDH2), serine/threonine protein phosphatase, (6)-methylguanine methyltransferase (MGMT) or multidrug resistance protein 1 (MDR1).

Said polynucleotide introduction step can be simultaneous, before or after the introduction or expression of said rare-cutting endonuclease. Depending on the location of the target nucleic acid sequence(s) wherein break event has occurred, such exogenous nucleic acid(s) can be used to knockout a gene, e.g. when exogenous nucleic acid(s) is located within the open reading frame of said gene, or to introduce new sequences or genes of interest. Sequence insertions by using such exogenous nucleic acid(s) can be used to modify a targeted existing gene, by correction or replacement of said gene (allele swap as a non-limiting example), or to up- or down-regulate the expression of the targeted gene (promoter swap as non-limiting example), said targeted gene correction or replacement.

### Antigen-presenting cells (APCs)

By "APCs cells" is meant the professional APCs i.e. those who express MHC class II molecules such the dendritic cells (DCs), macrophages, some B-cells and some activated epithelial cells. By "aAPCs cells" is meant synthetic versions of these APCs and are made by attaching the specific T-cell stimulating signals to various macro and micro biocompatible surfaces.

The present disclosure relates also to a method for producing antigen-presenting cell (APC) comprising the steps of:
a) transfecting a hematopoietic cell according to the transfection method such as described earlier, with bio-NPs nanoparticles coated with nucleic acids encoding an antigen;
b) after incubation, selecting the cells presenting said antigen at their surface.

According to one example, said bio-NPs nanoparticles having additionally targeting peptides/ligands to target them to said APC.

Said nucleic acid(s) to be expressed by APC may encode an antigen or a CAR.

According to a further example, the method for stimulating the antigen presentation by antigen-presenting cell (APC) is followed by a step of purification/enrichment.

The present disclosure encompassed also a method for generating artificial antigen-presenting cells (AAPCs) by transfecting antigen-presenting cells (APCs), which comprises contacting said APCs with nanoparticles having (entrapped or encapsulated) nucleic acid(s) to be expressed by said APC; said nanoparticles being additionally incubated with a targeting peptide/ligand to target them to said APCs.

### Transfected immune cell

The present invention relates to transfected immune cell obtained by the transfection method of the invention as described earlier, said cell comprising a silica-based nanoparticle coated with a nucleic acid, which comprises a heterologous sequence encoding a rare-cutting endonuclease, to be expressed into the cell or inserted into its genome.

The particularity of said transfected immune cell obtained by such gene delivery protocol can be found in a longer expression window compared to immune cell transfected by non-nanoparticles techniques.

In addition, transfected immune cell of the invention treated by such method of gene delivery display a higher rate of targeted integration compared to immune cell transfected by non-nanoparticles techniques.

In the scope of the present invention is also encompassed an isolated transfected immune cell, preferably a T-cell obtained according to the transfection method of the invention as previously described. Said immune cell refers to a cell of hematopoietic origin functionally involved in the initiation and/or execution of innate and/or adaptative immune response. Said immune cell according to the present invention can be derived from a stem cell. The stem cells can be adult stem cells, non-human embryonic stem cells, more particularly non-human stem cells, cord blood stem cells, progenitor cells, bone marrow stem cells, induced pluripotent stem cells, totipotent stem cells or hematopoietic stem cells. Representative human cells are CD34+ cells. Said isolated cell can also be a dendritic cell, killer dendritic cell, a mast cell, a NK-cell, a B-cell or a T-cell selected from the group consisting of inflammatory T-lymphocytes, cytotoxic T-lymphocytes, regulatory T-lymphocytes or helper T-lymphocytes. In another embodiment, said cell can be derived from the group consisting of CD4+ T-lymphocytes and CD8+ T-lymphocytes. Prior to expansion and genetic modification of the cells of the invention, a source of cells can be obtained from a subject through a variety of non-limiting methods. Cells can be obtained from a number of non-limiting sources, including peripheral blood mononuclear cells, bone marrow, lymph node tissue, cord blood, thymus tissue, tissue from a site of infection, ascites, pleural effusion, spleen tissue, and tumors. In certain embodiments of the present invention, any number of T cell lines available and known to those skilled in the art, may be used. In another embodiment, said cell can be derived from a healthy donor, from a patient diagnosed with cancer or from a patient diagnosed with an infection. In another embodiment, said cell is part of a mixed population of cells which present different phenotypic characteristics. In the scope of the present invention is also encompassed a cell line obtained from a transformed T- cell according to the method of the invention as previously described. Modified cells resistant to an immunosuppressive treatment and susceptible to be obtained by the previous method are encompassed in the scope of the present invention.

### Activation and expansion of T cells

Whether prior to or after genetic modification of the T cells, even if the genetically modified immune cells of the present invention are activated and proliferate independently of antigen binding mechanisms, the immune cells, particularly T-cells of the present invention can be further activated and expanded generally using methods as described, for example, in U.S. Patents 6,352,694; 6,534,055; 6,905,680; 6,692,964; 5,858,358; 6,887,466; 6,905,681; 7,144,575; 7,067,318; 7,172,869; 7,232,566; 7,175,843; 5,883,223; 6,905,874; 6,797,514; 6,867,041; and U.S. Patent Application Publication No. 20060121005. T cells can be expanded *in vitro.*

Generally, the T cells of the invention are expanded by contact with an agent that stimulates a CD3 TCR complex and a co-stimulatory molecule on the surface of the T cells to create an activation signal for the T-cell. For example, chemicals such as calcium ionophore A23187, phorbol 12-myristate 13-acetate (PMA), or mitogenic lectins like phytohemagglutinin (PHA) can be used to create an activation signal for the T-cell.

As non-limiting examples, T cell populations may be stimulated *in vitro* such as by contact with an anti-CD3 antibody, or antigen-binding fragment thereof, or an anti-CD2 antibody immobilized on a surface, or by contact with a protein kinase C activator (e.g., bryostatin) in conjunction with a calcium ionophore. For co-stimulation of an accessory molecule on the surface of the T cells, a ligand that binds the accessory molecule is used. For example, a population of T cells can be contacted with an anti-CD3 antibody and an anti-CD28 antibody, under conditions appropriate for stimulating proliferation of the T cells. Conditions appropriate for T cell culture include an appropriate media (e.g., Minimal Essential Media or RPMI Media 1640 or, X-vivo 5, (Lonza)) that may contain factors necessary for proliferation and viability, including serum (e.g., fetal bovine or human serum), interleukin-2 (IL-2), insulin, IFN-g , 1L-4, 1L-7, GM-CSF, -10, - 2, 1L-15, TGFp, and TNF- or any other additives for the growth of cells known to the skilled artisan. Other additives for the growth of cells include, but are not limited to, surfactant, plasmanate, and reducing agents such as N-acetyl-cysteine and 2-mercaptoethanoi. Media can include RPMI 1640, A1M-V, DMEM, MEM, a-MEM, F-12, X-Vivo 1, and X-Vivo 20, Optimizer, with added amino acids, sodium pyruvate, and vitamins, either serum-free or supplemented with an appropriate amount of serum (or plasma) or a defined set of hormones, and/or an amount of cytokine(s) sufficient for the growth and expansion of T cells. Antibiotics, e.g., penicillin and streptomycin, are included only in experimental cultures, not in cultures of cells that are to be infused into a subject. The target cells are maintained under conditions necessary to support growth, for example, an appropriate temperature (e.g., 37° C) and atmosphere (e.g., air plus 5% C02). T cells that have been exposed to varied stimulation times may exhibit different characteristics

In another particular embodiment, said cells can be expanded by co-culturing with tissue or cells.

### Pharmaceutical composition containing transfected immune cells

Also in the scope of the present invention, a pharmaceutical composition comprising the transfected immune cells obtained by as earlier explained and optionally a pharmaceutically acceptable carrier and/or diluent.

### Other definitions

- Amino acid residues in a polypeptide sequence are designated herein according to the one-letter code, in which, for example, Q means Gln or Glutamine residue, R means Arg or Arginine residue and D means Asp or Aspartic acid residue.
- Amino acid substitution means the replacement of one amino acid residue with another, for instance the replacement of an Arginine residue with a Glutamine residue in a peptide sequence is an amino acid substitution.
- Nucleotides are designated as follows: one-letter code is used for designating the base of a nucleoside: a is adenine, t is thymine, c is cytosine, and g is guanine. For the degenerated nucleotides, r represents g or a (purine nucleotides), k represents g or t, s represents g or c, w represents a or t, m represents a or c, y represents t or c (pyrimidine nucleotides), d represents g, a or t, v represents g, a or c, b represents g, t or c, h represents a, t or c, and n represents g, a, t or c.
- "As used herein, "nucleic acid" or "polynucleotides" refers to nucleotides and/or polynucleotides, such as deoxyribonucleic acid (DNA) or ribonucleic acid (RNA), oligonucleotides, fragments generated by the polymerase chain reaction (PCR), and fragments generated by any of ligation, scission, endonuclease action, and exonuclease action. Nucleic acid molecules can be composed of monomers that are naturally-occurring nucleotides (such as DNA and RNA), or analogs of naturally-occurring nucleotides (e.g., enantiomeric forms of naturally-occurring nucleotides), or a combination of both. Modified nucleotides can have alterations in sugar moieties and/or in pyrimidine or purine base moieties. Sugar modifications include, for example, replacement of one or more hydroxyl groups with halogens, alkyl groups, amines, and azido groups, or sugars can be functionalized as ethers or esters. Moreover, the entire sugar moiety can be replaced with sterically and electronically similar structures, such as aza-sugars and carbocyclic sugar analogs. Examples of modifications in a base moiety include alkylated purines and pyrimidines, acylated purines or pyrimidines, or other well-known heterocyclic substitutes. Nucleic acid monomers can be linked by phosphodiester bonds or analogs of such linkages. Nucleic acids can be either single stranded or double stranded.
- By chimeric antigen receptor (CAR) is intended molecules that combine a binding domain against a component present on the target cell, for example an antibody-based specificity for a desired antigen (e.g., tumor antigen) with a T cell receptor-activating intracellular domain to generate a chimeric protein that exhibits a specific anti-target cellular immune activity. Generally, CAR consists of an extracellular single chain antibody (scFvFc), fused to the intracellular signaling domain of the T cell antigen receptor complex zeta chain (scFvFc:ζ) and have the ability, when expressed in T cells, to redirect antigen recognition based on the monoclonal antibody's specificity. CAR may sometimes comprise multiple transmembrane polypeptides (multi-chain CARs) as described in WO2014039523. One example of CAR used in the present invention is a CAR directing against 5T4 antigen and can comprise as non-limiting example the amino acid sequences : SEQ ID NO: 19 to 42.
- The term "endonuclease" refers to any wild-type or variant enzyme capable of catalyzing the hydrolysis (cleavage) of bonds between nucleic acids within a DNA or RNA molecule, preferably a DNA molecule. Endonucleases do not cleave the DNA or RNA molecule irrespective of its sequence, but recognize and cleave the DNA or RNA molecule at specific polynucleotide sequences, further referred to as "target sequences" or "target sites". Endonucleases can be classified as rare-cutting endonucleases when having typically a polynucleotide recognition site greater than 12 base pairs (bp) in length, more preferably of 14-55 bp. Rare-cutting endonucleases significantly increase HR by inducing DNA double-strand breaks (DSBs) at a defined locus (Perrin, Buckle et al. 1993; Rouet, Smih et al. 1994; Choulika, Perrin et al. 1995; Pingoud and Silva 2007). Rare-cutting endonucleases can for example be a homing endonuclease (Paques and Duchateau 2007), a chimeric Zinc-Finger nuclease (ZFN) resulting from the fusion of engineered zinc-finger domains with the catalytic domain of a restriction enzyme such as Fokl (Porteus and Carroll 2005), a Cas9 endonuclease from CRISPR system (Gasiunas, Barrangou et al. 2012; Jinek, Chylinski et al. 2012; Cong, Ran et al. 2013; Mali, Yang et al. 2013) or a chemical endonuclease (Eisenschmidt, Lanio et al. 2005; Arimondo, Thomas et al. 2006). In chemical endonucleases, a chemical or peptidic cleaver is conjugated either to a polymer of nucleic acids or to another DNA recognizing a specific target sequence, thereby targeting the cleavage activity to a specific sequence. Chemical endonucleases also encompass synthetic nucleases like conjugates of orthophenanthroline, a DNA cleaving molecule, and triplex-forming oligonucleotides (TFOs), known to bind specific DNA sequences (Kalish and Glazer 2005). Such chemical endonucleases are comprised in the term "endonuclease" according to the present invention.
- By a "TALE-nuclease" (TALEN) is intended a fusion protein consisting of a nucleic acid-binding domain typically derived from a Transcription Activator Like Effector (TALE) and one nuclease catalytic domain to cleave a nucleic acid target sequence. The catalytic domain is preferably a nuclease domain and more preferably a domain having endonuclease activity, like for instance I-Tevl, ColE7, NucA and Fok-I. In a particular embodiment, the TALE domain can be fused to a meganuclease like for instance I-Crel and I-Onul or functional variant thereof. In a more preferred embodiment, said nuclease is a monomeric TALE-Nuclease. A monomeric TALE-Nuclease is a TALE-Nuclease that does not require dimerization for specific recognition and cleavage, such as the fusions of engineered TAL repeats with the catalytic domain of I-Tevl described in WO2012138927. Transcription Activator like Effector (TALE) are proteins from the bacterial species *Xanthomonas* comprise a plurality of repeated sequences, each repeat comprising di-residues in position 12 and 13 (RVD) that are specific to each nucleotide base of the nucleic acid targeted sequence. Binding domains with similar modular base-per-base nucleic acid binding properties (MBBBD) can also be derived from new modular proteins recently discovered by the applicant in a different bacterial species. The new modular proteins have the advantage of displaying more sequence variability than TAL repeats. Preferably, RVDs associated with recognition of the different nucleotides are HD for recognizing C, NG for recognizing T, NI for recognizing A, NN for recognizing G or A, NS for recognizing A, C, G or T, HG for recognizing T, IG for recognizing T, NK for recognizing G, HA for recognizing C, ND for recognizing C, HI for recognizing C, HN for recognizing G, NA for recognizing G, SN for recognizing G or A and YG for recognizing T, TL for recognizing A, VT for recognizing A or G and SW for recognizing A. In another embodiment, critical amino acids 12 and 13 can be mutated towards other amino acid residues in order to modulate their specificity towards nucleotides A, T, C and G and in particular to enhance this specificity. TALE-nuclease have been already described and used to stimulate gene targeting and gene modifications (Boch, Scholze et al. 2009; Moscou and Bogdanove 2009; Christian, Cermak et al. 2010; Li, Huang et al. 2011). Custom-made TAL-nucleases are commercially available under the trade name TALEN^{™} (Cellectis, 8 rue de la Croix Jarry, 75013 Paris, France).

The rare-cutting endonuclease according to the present invention can also be a Cas9 endonuclease. Recently, a new genome engineering tool has been developed based on the RNA-guided Cas9 nuclease (Gasiunas, Barrangou et al. 2012; Jinek, Chylinski et al. 2012; Cong, Ran et al. 2013; Mali, Yang et al. 2013) from the type II prokaryotic CRISPR (Clustered Regularly Interspaced Short palindromic Repeats) adaptive immune system (see for review (Sorek, Lawrence et al. 2013)). The CRISPR Associated (Cas) system was first discovered in bacteria and functions as a defense against foreign DNA, either viral or plasmid. CRISPR-mediated genome engineering first proceeds by the selection of target sequence often flanked by a short sequence motif, referred as the proto-spacer adjacent motif (PAM). Following target sequence selection, a specific crRNA, complementary to this target sequence is engineered. Trans-activating crRNA (tracrRNA) required in the CRISPR type II systems paired to the crRNA and bound to the provided Cas9 protein. Cas9 acts as a molecular anchor facilitating the base pairing of tracRNA with cRNA (Deltcheva, Chylinski et al. 2011). In this ternary complex, the dual tracrRNA:crRNA structure acts as guide RNA that directs the endonuclease Cas9 to the cognate target sequence. Target recognition by the Cas9-tracrRNA:crRNA complex is initiated by scanning the target sequence for homology between the target sequence and the crRNA. In addition to the target sequence-crRNA complementarity, DNA targeting requires the presence of a short motif adjacent to the protospacer (protospacer adjacent motif - PAM). Following pairing between the dual-RNA and the target sequence, Cas9 subsequently introduces a blunt double strand break 3 bases upstream of the PAM motif (Garneau, Dupuis et al. 2010).

Rare-cutting endonuclease can be a homing endonuclease, also known under the name of meganuclease. Such homing endonucleases are well-known to the art (Stoddard 2005). Homing endonucleases recognize a DNA target sequence and generate a single- or double-strand break. Homing endonucleases are highly specific, recognizing DNA target sites ranging from 12 to 45 base pairs (bp) in length, usually ranging from 14 to 40 bp in length. The homing endonuclease according to the invention may for example correspond to a LAGLIDADG endonuclease, to a HNH endonuclease, or to a GIY-YIG endonuclease. Preferred homing endonuclease according to the present invention can be an I*-Crel* variant.
- by "mutation" is intended the substitution, deletion, insertion of up to one, two, three, four, five, six, seven, eight, nine, ten, eleven, twelve, thirteen, fourteen, fifteen, twenty, twenty five, thirty, fourty, fifty, or more nucleotides/amino acids in a polynucleotide (cDNA, gene) or a polypeptide sequence. The mutation can affect the coding sequence of a gene or its regulatory sequence. It may also affect the structure of the genomic sequence or the structure/stability of the encoded mRNA.
- by "variant(s)", it is intended a repeat variant, a variant, a DNA binding variant, a TALE-nuclease variant, a polypeptide variant obtained by mutation or replacement of at least one residue in the amino acid sequence of the parent molecule.
- by "functional variant" is intended a catalytically active mutant of a protein or a protein domain; such mutant may have the same activity compared to its parent protein or protein domain or additional properties, or higher or lower activity.
- "identity" refers to sequence identity between two nucleic acid molecules or polypeptides. Identity can be determined by comparing a position in each sequence which may be aligned for purposes of comparison. When a position in the compared sequence is occupied by the same base, then the molecules are identical at that position. A degree of similarity or identity between nucleic acid or amino acid sequences is a function of the number of identical or matching nucleotides at positions shared by the nucleic acid sequences. Various alignment algorithms and/or programs may be used to calculate the identity between two sequences, including FASTA, or BLAST which are available as a part of the GCG sequence analysis package (University of Wisconsin, Madison, Wis.), and can be used with, e.g., default setting. For example, polypeptides having at least 70%, 85%, 90%, 95%, 98% or 99% identity to specific polypeptides described herein and preferably exhibiting substantially the same functions, as well as polynucleotide encoding such polypeptides, are contemplated. Unless otherwise indicated a similarity score will be based on use of BLOSUM62. When BLASTP is used, the percent similarity is based on the BLASTP positives score and the percent sequence identity is based on the BLASTP identities score. BLASTP "Identities" shows the number and fraction of total residues in the high scoring sequence pairs which are identical; and BLASTP "Positives" shows the number and fraction of residues for which the alignment scores have positive values and which are similar to each other. Amino acid sequences having these degrees of identity or similarity or any intermediate degree of identity of similarity to the amino acid sequences disclosed herein are contemplated and encompassed by this disclosure. The polynucleotide sequences of similar polypeptides are deduced using the genetic code and may be obtained by conventional means, in particular by reverse translating its amino acid sequence using the genetic code.
- "signal-transducing domain" or "co-stimulatory ligand" refers to a molecule on an antigen presenting cell that specifically binds a cognate co-stimulatory molecule on a T-cell, thereby providing a signal which, in addition to the primary signal provided by, for instance, binding of a TCR/CD3 complex with an MHC molecule loaded with peptide, mediates a T cell response, including, but not limited to, proliferation activation, differentiation and the like. A co-stimulatory ligand can include but is not limited to CD7, B7-1 (CD80), B7-2 (CD86), PD-L1, PD-L2, 4-1BBL, OX40L, inducible costimulatory igand (ICOS-L), intercellular adhesion molecule (ICAM, CD30L, CD40, CD70, CD83, HLA-G, MICA, M1CB, HVEM, lymphotoxin beta receptor, 3/TR6, ILT3, ILT4, an agonist or antibody that binds Toll ligand receptor and a ligand that specifically binds with B7-H3. A co-stimulatory ligand also encompasses, inter alia, an antibody that specifically binds with a co-stimulatory molecule present on a T cell, such as but not limited to, CD27, CD28, 4-IBB, OX40, CD30, CD40, PD-1, ICOS, lymphocyte function-associated antigen-1 (LFA-1), CD2, CD7, LTGHT, NKG2C, B7-H3, a ligand that specifically binds with CD83.

A "co-stimulatory molecule" refers to the cognate binding partner on a Tcell that specifically binds with a co-stimulatory ligand, thereby mediating a co-stimulatory response by the cell, such as, but not limited to proliferation. Co-stimulatory molecules include, but are not limited to an MHC class I molecule, BTLA and Toll ligand receptor.

A "co-stimulatory signal" as used herein refers to a signal, which in combination with primary signal, such as TCR/CD3 ligation, leads to T cell proliferation and/or upregulation or downregulation of key molecules.

The term "extracellular ligand-binding domain" as used herein is defined as an oligo- or polypeptide that is capable of binding a ligand. Preferably, the domain will be capable of interacting with a cell surface molecule. For example, the extracellular ligand-binding domain may be chosen to recognize a ligand that acts as a cell surface marker on target cells associated with a particular disease state. Thus examples of cell surface markers that may act as ligands include those associated with viral, bacterial and parasitic infections, autoimmune disease and cancer cells.

The term "subject" or "patient" as used herein includes all members of the animal kingdom including non-human primates and humans.

The above written description of the invention provides a manner and process of making and using it such that any person skilled in this art is enabled to make and use the same, this enablement being provided in particular for the subject matter of the appended claims, which make up a part of the original description.

Where a numerical limit or range is stated herein, the endpoints are included. Also, all values and subranges within a numerical limit or range are specifically included as if explicitly written out.

Having generally described this invention, a further understanding can be obtained by reference to certain specific examples, which are provided herein for purposes of illustration only, and are not intended to be limiting unless otherwise specified.

### Examples

### Example1: Evaluation of the nanoparticle-biomolecule conjugates for their efficacy, long term expression and toxicity

A dose range of nanoparticle-biomolecule conjugates containing either mRNA or DNA allowing GFP expression (SEQ ID N°1), is incubated with T cells. At different time point post incubation T cells are harvested to determinate by flow cytometry their viability, the percentage of T cells expressing GFP (efficacy) and the GFP expression intensity. These results aim to show that the nanoparticles are able to deliver acid nucleic resulting in GFP expression.

This should allow the determination of the optimal dose in order to get the highest efficacy with the lowest toxicity.

### Example2: Targeted integration induced by TALE-nuclease electroporation and targeting vector delivery by nanoparticle-biomolecule conjugates

Different doses of nanoparticle-biomolecule conjugates containing the pSel-EF1 DNA vector (SEQID N°2) designed for homologous recombination at the TRAC locus (Figure 4) are incubated with T cells. At different time points after nanoparticles incubation, the couple of mRNAs encoding TALE-nuclease targeting TRAC locus (SEQ ID N°3-4, left and right TALEN respectively) are electroporated using AgilPulse technology according to the manufacturer protocol. T cells are harvested at different time-points in order to determine the percentage of homologous recombination events by measuring the percentage of GFP+ cells using flow-cytometry.

The results aim to show that the combination of nanoparticle-biomolecule conjugates for delivery of the targeting vector and the mRNA for delivery of the mRNA encoding the TALE-nuclease are able to induce homologous recombination in T cells.

### Example3: Targeted integration induced by TALE-nuclease and targeting vector delivered both by nanoparticle-biomolecule conjugates

Different doses of nanoparticle-biomolecule conjugates containing the DNA vector (SEQ ID N°2) and the couple of mRNAs encoding the TALE-nuclease targeting TRAC locus (SEQ ID N°3-4, left and right TALEN respectively) are incubated with T cells. At different time-points post nanoparticles incubation, T cells are harvested in order to determine the percentage of homologous recombination events by measuring the percentage of GFP+ cells using flow-cytometry.

The results show that nanoparticle-biomolecule conjugates containing both the targeting vector and the mRNA to encoding the TALE-nuclease targeting TRAC locus are able to induce homologous recombination in T cells.

## Claims

1. A method of transfecting a primary hematopoietic cell with nucleic acids, at least one which encodes for a rare-cutting endonuclease, to be expressed into said cell or to be introduced into its genome, said method comprising the steps of:
a) isolating hematopoietic cells;
b) culturing the hematopoietic cells in a condition where they can expand;
c) loading nanoparticle-biomolecule conjugates with nucleic acids, at least one which encodes a rare-cutting endonuclease, to be expressed into said cell or to be introduced into its genome;
d) incubating said hematopoietic cells with said nanoparticle-biomolecule conjugates to have them penetrate the cells;
wherein said nanoparticle-biomolecule conjugates are silica-based nanoparticles which are coated with cell penetrating peptides (CPPs) and/or ligand targeting molecule.

2. Method according to claim 1, wherein said silica-based nanoparticles are mesoporous.

3. Method according to claim 1 to 2, wherein said nanoparticle-biomolecule conjugates comprise at least 1,2- dioleoyl-3-trimethylammonium-propane (DOTAP), 1,2-ditetradecanoyl-sn-glycero-3- phosphocholine (DMPC), polyethylene glycol (PEG), and cholesterol.

4. Method according to any one of claims 1 to 3, wherein said nucleic acid is encapsulated into porous silica nanoparticle-supported lipid bilayers.

5. Method according to any one of claims 1 to 4, wherein said nanoparticle biomolecule conjugates are multilayered.

6. Method according to claim 5, wherein said nucleic acid is contained in the core of said multilayered nanoparticles.

7. Method according to claim 6, wherein one core-stabilizing interlayer of said nanoparticles comprises at least silica, chitosan, polyε-caprolactone or polyphosphoramidate (PPA).

8. Method according to claim 7, wherein one inner endosome-disrupting interlayer is present on the top of said core-stabilizing interlayer, said interlayer comprising at least a polycation, such as polylysine, polyarginine, protamine, polyethylenimine or histone.

9. Method according to claim 8, wherein the outer layer comprises at least a biocompatible polymer, such as polyethylene glycol PEG.

10. Method according to any one of claims 1 to 9, wherein said nanoparticles have one layer comprising a polyanion, such as poly(D-glutamic acid).

11. Method according to any one of claim 1 to 10, wherein at least one of said nucleic acids is tagged by a nuclear localization sequence (NLS).

12. Method according to any one of claims 1 to 11, wherein it further comprises the step of:
e) purifying the hematopoietic cells which have expressed said heterologous nucleic sequence and/or integrated it into their genome.

13. Method according to any one of claim 1 to 12, wherein at least one of said nucleic acids encodes for a chimeric antigen receptor (CAR) or a multi-chain CAR.

14. Method according to anyone of claims 1 to 13, wherein said rare-cutting endonuclease is Cas9, Cpf1, Argonaute, TALEN, ZFN or a homing endonuclease.

15. Method according to any one of claim 1 to 14, wherein said nanoparticle-biomolecule conjugates are obtained by admixing an sgRNA and Cas9 protein mixture with a mixture comprising or consisting of surfactant, phospholipid, biodegradable polymer, lipoprotein or alcohol.

16. Method according to any one of claims 1 to 15, wherein at least one of said nucleic acids is a DNA matrix that can be integrated through non-homologous end joining (NHEJ) or homologous recombination (HR) at a genome site.

17. Method according to any one of claim 1 to 16, wherein said hematopoietic cells are T-cells.

18. Method according to claim 17, wherein said CAR or multi-chain CAR is specific to a cell surface antigen chosen amongst CD38, CD123 or CS1.

19. Method according to any one of claims 1 to 18, wherein said hematopoietic cells were previously isolated from donors or a patient.

20. A transfected hematopoietic cell, which is obtainable according to the method of any one of claims 1 to 19, said cell comprising a silica-based nanoparticle coated with cell penetrating peptides (CPPs) and/or ligand targeting molecule and further coated with a nucleic acid, which comprises a heterologous sequence encoding a rare-cutting endonuclease, to be expressed into the cell or inserted into its genome.

21. A hematopoietic cell according to claim 20 for its use for the treatment of infected or malignant cells.

22. A pharmaceutical composition comprising a transfected hematopoietic cell according to claim 20 or 21, and optionally a pharmaceutically acceptable carrier and/or diluent.

## Patentansprüche

1. Verfahren zum Transfizieren einer primären hämatopoetischen Zelle mit Nukleinsäuren, von denen mindestens eine für eine selten schneidende Endonuklease kodiert, die in die Zelle zu exprimieren oder in ihr Genom einzuführen ist, wobei das Verfahren die folgenden Schritte umfasst:
a) Isolieren von hämatopoetischen Zellen;
b) Kultivieren der hämatopoetischen Zellen in einem Zustand, in dem sie sich ausbreiten können;
c) Laden von Nanopartikel-Biomolekül-Konjugaten mit Nukleinsäuren, von denen mindestens eine für eine selten schneidende Endonuklease kodiert, die in die Zelle zu exprimieren oder in ihr Genom einzuführen ist;
d) Inkubieren der hämatopoetischen Zellen mit den Nanopartikel-Biomolekül-Konjugaten, damit diese die Zellen penetrieren;
wobei die Nanopartikel-Biomolekül-Konjugate Nanopartikel auf Siliziumdioxidbasis sind, die mit zellpenetrierenden Peptiden (CPPs) und/oder einem Ligandenzielmolekül beschichtet sind.

2. Verfahren nach Anspruch 1, wobei die Nanopartikel auf Siliziumdioxidbasis mesoporös sind.

3. Verfahren nach Anspruch 1 bis 2, wobei die Nanopartikel-Biomolekül-Konjugate mindestens 1,2-Dioleoyl-3-trimethylammonium-propan (DOTAP), 1,2-Ditetradecanoyl-sn-glycero-3-phosphocholin (DMPC), Polyethylenglycol (PEG) und Cholesterin umfassen.

4. Verfahren nach einem der Ansprüche 1 bis 3, wobei die Nukleinsäure in poröse, von Siliziumdioxid-Nanopartikeln gestützte Lipid-Doppelschichten eingekapselt wird.

5. Verfahren nach einem der Ansprüche 1 bis 4, wobei die Nanopartikel-Biomolekül-Konjugate mehrschichtig sind.

6. Verfahren nach Anspruch 5, wobei die Nukleinsäure in dem Kern der mehrschichtigen Nanopartikel enthalten ist.

7. Verfahren nach Anspruch 6, wobei eine kernstabilisierende Zwischenschicht der Nanopartikel mindestens Siliziumdioxid, Chitosan, Polycaprolacton oder Polyphosphorsäureamidat (PPA) umfasst.

8. Verfahren nach Anspruch 7, wobei eine innere, das Endosom störende Zwischenschicht auf der kernstabilisierenden Zwischenschicht vorhanden ist, wobei die Zwischenschicht mindestens ein Polykation, wie etwa Polylysin, Polyarginin, Protamin, Polyethylenimin oder Histon, umfasst.

9. Verfahren nach Anspruch 8, wobei die Außenschicht mindestens ein biokompatibles Polymer, wie etwa Polyethylenglycol (PEG), umfasst.

10. Verfahren nach einem der Ansprüche 1 bis 9, wobei die Nanopartikel eine Schicht aufweisen, die ein Polyanion, wie etwa Poly(D-glutaminsäure), umfasst.

11. Verfahren nach einem der Ansprüche 1 bis 10, wobei mindestens eine der Nukleinsäuren durch eine Kernlokalisierungssequenz (NLS) getaggt ist.

12. Verfahren nach einem der Ansprüche 1 bis 11, wobei es ferner den folgenden Schritt umfasst:
e) Reinigen der hämatopoetischen Zellen, welche die heterologe Nukleinsequenz exprimiert und/oder diese in ihr Genom integriert haben.

13. Verfahren nach einem der Ansprüche 1 bis 12, wobei mindestens eine der Nukleinsäuren für einen chimären Antigenrezeptor (CAR) oder einen mehrkettigen CAR kodiert.

14. Verfahren nach einem der Ansprüche 1 bis 13, wobei es sich bei der selten schneidenden Endonuklease um Cas9, Cpf1, Argonaute, TALEN, ZFN oder eine Homing-Endonuklease handelt.

15. Verfahren nach einem der Ansprüche 1 bis 14, wobei die Nanopartikel-Biomolekül-Konjugate durch Beimischen eines sgRNA- und Cas9-Proteingemischs mit einem Gemisch erhalten werden, das ein Tensid, Phospholipid, biologisch abbaubares Polymer, Lipoprotein oder Alkohol umfasst oder daraus besteht.

16. Verfahren nach einem der Ansprüche 1 bis 15, wobei es sich bei mindestens einer der Nukleinsäuren um eine DNA-Matrix handelt, die durch eine nichthomologe Endverknüpfung (NHEJ) oder eine homologe Rekombination (HR) an einer Genomstelle integriert werden kann.

17. Verfahren nach einem der Ansprüche 1 bis 16, wobei es sich bei den hämatopoetischen Zellen um T-Zellen handelt.

18. Verfahren nach Anspruch 17, wobei der CAR oder der mehrkettige CAR spezifisch für ein Zelloberflächenantigen ist, das aus CD38, CD123 oder CS1 ausgewählt wird.

19. Verfahren nach einem der Ansprüche 1 bis 18, wobei die hämatopoetischen Zellen zuvor von Spendern oder einem Patienten isoliert wurden.

20. Transfizierte hämatopoetische Zelle, die nach dem Verfahren nach einem der Ansprüche 1 bis 19 erhaltbar ist, wobei die Zelle einen Nanopartikel auf Siliziumdioxidbasis umfasst, der mit zellpenetrierenden Peptiden (CPPs) und/oder einem Ligandenzielmolekül beschichtet ist und ferner mit einer Nukleinsäure beschichtet ist, die eine heterologe Sequenz umfasst, die für eine selten schneidende Endonuklease kodiert, die in der Zelle zu exprimieren oder in ihr Genom zu insertieren ist.

21. Hämatopoetische Zelle nach Anspruch 20 zur Verwendung für die Behandlung von infizierten oder malignen Zellen.

22. Pharmazeutische Zusammensetzung, umfassend eine transfizierte hämatopoetische Zelle nach Anspruch 20 oder 21 und gegebenenfalls einen pharmazeutisch unbedenklichen Träger und/oder ein Verdünnungsmittel.

## Revendications

1. Procédé de transfection d'une cellule hématopoïétique primaire avec des acides nucléiques, dont au moins un code pour une endonucléase à coupure rare, à exprimer dans ladite cellule ou à introduire dans son génome, ledit procédé comprenant les étapes consistant à :
a) isoler des cellules hématopoïétiques ;
b) cultiver les cellules hématopoïétiques dans un état où elles peuvent se développer ;
c) charger des conjugués nanoparticule-biomolécule avec des acides nucléiques, dont au moins un code pour une endonucléase à coupure rare, à exprimer dans ladite cellule ou à introduire dans son génome ;
d) incuber lesdites cellules hématopoïétiques avec lesdits conjugués nanoparticule-biomolécule pour les faire pénétrer dans les cellules ;
lesdits conjugués nanoparticule-biomolécule étant des nanoparticules à base de silice qui sont revêtues de peptides pénétrant dans les cellules (CPP) et/ou d'une molécule de ciblage de ligand.

2. Procédé selon la revendication 1, dans lequel lesdites nanoparticules à base de silice sont mésoporeuses.

3. Procédé selon la revendication 1 à 2, dans lequel lesdits conjugués nanoparticule-biomolécule comprennent au moins le 1,2-dioléoyl-3-triméthylammonium-propane (DOTAP), le 1,2-ditétradécanoyl-sn-glycéro-3-phosphocholine (DMPC), le polyéthylène glycol (PEG) et le cholestérol.

4. Procédé selon l'une quelconque des revendications 1 à 3, dans lequel ledit acide nucléique est encapsulé dans des bicouches lipidiques supportées par des nanoparticules de silice poreuses.

5. Procédé selon l'une quelconque des revendications 1 à 4, dans lequel lesdits conjugués nanoparticule-biomolécule sont multicouches.

6. Procédé selon la revendication 5, dans lequel ledit acide nucléique est contenu dans le noyau desdites nanoparticules multicouches.

7. Procédé selon la revendication 6, dans lequel une couche intermédiaire stabilisatrice de noyau desdites nanoparticules comprend au moins la silice, le chitosane, le polyε-caprolactone ou le polyphosphoramidate (PPA).

8. Procédé selon la revendication 7, dans lequel une couche intermédiaire perturbatrice d'endosome interne est présente sur le dessus de ladite couche intermédiaire stabilisatrice de noyau, ladite couche intermédiaire comprenant au moins un polycation, tel que la polylysine, la polyarginine, la protamine, la polyéthylèneimine ou l'histone.

9. Procédé selon la revendication 8, dans lequel la couche externe comprend au moins un polymère biocompatible, tel que le polyéthylène glycol PEG.

10. Procédé selon l'une quelconque des revendications 1 à 9, dans lequel lesdites nanoparticules ont une couche comprenant un polyanion, tel que l'acide poly(D-glutamique).

11. Procédé selon l'une quelconque des revendications 1 à 10, dans lequel au moins un desdits acides nucléiques est marqué par une séquence de localisation nucléaire (NLS).

12. Procédé selon l'une quelconque des revendications 1 à 11, dans lequel il comprend en outre l'étape consistant à :
e) purifier les cellules hématopoïétiques qui ont exprimé ladite séquence nucléique hétérologue et/ou l'ont intégrée dans leur génome.

13. Procédé selon l'une quelconque des revendications 1 à 12, dans lequel au moins un desdits acides nucléiques code pour un récepteur antigénique chimérique (CAR) ou un CAR multicaténaire.

14. Procédé selon l'une quelconque des revendications 1 à 13, dans lequel ladite endonucléase à coupure rare est Cas9, Cpf1, Argonaute, TALEN, ZFN ou une endonucléase homing.

15. Procédé selon l'une quelconque des revendications 1 à 14, dans lequel lesdits conjugués nanoparticule-biomolécule sont obtenus en mélangeant un mélange de sgRNA et de protéine Cas9 avec un mélange comprenant ou constitué de tensioactif, de phospholipide, de polymère biodégradable, de lipoprotéine ou d'alcool.

16. Procédé selon l'une quelconque des revendications 1 à 15, dans lequel au moins un desdits acides nucléiques est une matrice d'ADN qui peut être intégrée par jonction d'extrémités non homologues (NHEJ) ou recombinaison homologue (HR) au niveau d'un site génomique.

17. Procédé selon l'une quelconque des revendications 1 à 16, dans lequel lesdites cellules hématopoïétiques sont des lymphocytes T.

18. Procédé selon la revendication 17, dans lequel ledit CAR ou CAR multicaténaire est spécifique d'un antigène de surface cellulaire choisi parmi CD38, CD123 ou CS1.

19. Procédé selon l'une quelconque des revendications 1 à 18, dans lequel lesdites cellules hématopoïétiques ont été préalablement isolées à partir de donneurs ou d'un patient.

20. Cellule hématopoïétique transfectée, qui peut être obtenue selon le procédé de l'une quelconque des revendications 1 à 19, ladite cellule comprenant une nanoparticule à base de silice revêtue de peptides pénétrant dans les cellules (CPP) et/ou d'une molécule de ciblage de ligand et revêtue en outre d'un acide nucléique, qui comprend une séquence hétérologue codant pour une endonucléase à coupure rare, à exprimer dans la cellule ou à insérer dans son génome.

21. Cellule hématopoïétique selon la revendication 20 pour son utilisation pour le traitement de cellules infectées ou malignes.

22. Composition pharmaceutique comprenant une cellule hématopoïétique transfectée selon la revendication 20 ou 21, et éventuellement un support et/ou un diluant pharmaceutiquement acceptable.
